# EUROPÄISCHE PATENTSCHRIFT

(11) **EP 0 791 343 B1**
(45) Veröffentlichungstag und Bekanntmachung des Hinweises auf die Patenterteilung: **08.05.2002**
(21) Anmeldenummer: 97102432.8
(22) Anmeldetag: 14.02.1997
(51) Int. Cl.: A61F 2/38

(54) **Kniegelenkendoprothese**
Knee joint endoprosthesis
Endoprothese de l'articulation du genou

(30) Priorität: 21.02.1996 DE 19606462
(43) Veröffentlichungstag der Anmeldung: 27.08.1997
(73) Patentinhaber: PLUS ENDOPROTHETIK AG, 6343 Rotkreuz (CH)
(72) Erfinder: Schmotzer, Hans, Dr., 5000 Aarau (CH); Horber, Willi, Dr., 8005 Zürich (CH)
(74) Vertreter: Popp, Eugen, Dr.

(56) Entgegenhaltungen:
- EP-A- 0 420 460
- EP-A- 0 627 203
- WO-A-90/11739
- DE-A- 2 539 717
- US-A- 3 969 773
- US-A- 4 655 778
- US-A- 5 370 701

## Beschreibung

Die Erfindung betrifft eine Kniegelenkendoprothese, mit einem Femurteil, das am unteren Ende eines Femurschaftes konvex gekrümmte Kondylenschalen mit ersten Gleitflächen aufweist, die dorsal zwei voneinander beabstandete, einen interkondylären Zwischenraum bildende Seitenwände besitzen, mit einem Tibiateil, das am oberen Ende eines Tibiaschaftes ein Tibiaplateau aufweist, auf dem den ersten Gleitflächen der Kondylenschalen entsprechende zweite Gleitflächen ausgebildet sind, und das eine sich axial in dem Tibiaschaft erstreckende Ausnehmung aufweist, und mit einem Kupplungsteil, das am oberen Ende eines drehbar in der Ausnehmung aufgenommenen Kupplungszapfens einen in den interkondylären Zwischenraum hineinragenden Gelenkkopf aufweist, der eine quer zur Femurachse verlaufende Bohrung besitzt und mittels eines durch die Bohrung laufenden und sich an den angrenzenden Seitenwänden der Kondylenschalen abstützenden Kupplungsbolzens schwenkbar gelagert ist.

Eine derartige Kniegelenkendoprothese mit einem Kupplungsteil in Form eines reinen Scharniergelenks ist aus der DE 35 29 894 C2 bekannt. Der Nachteil einer solchen Kniegelenkendoprothese besteht insbesondere darin, daß bei axialen Fehlstellungen zwischen Femur und Tibia keine Varus-/Valguskompensation möglich ist und die daraus resultierende einseitige Belastung vollständig von dem Kupplungsbolzen bzw. dessen Verankerung in den Seitenwänden aufgenommen werden muß. Da es aus Gewichtsersparnisgründen vielfach üblich ist, die beiden Seitenwände der Kondylenschalen mit einem Steg zu einem Kasten zu verbinden, an dem dann auch der Femurschaft ansetzt, sind auch Kastenbrüche bei fehlender Möglichkeit zur Varus-/Valguskompensation wohlbekannte Folgen.

Dieses Problem wurde bereits früh erkannt und der starre Kupplungsbolzen durch eine Kugel ersetzt, welche auf der Spitze eines vom Tibiateil ausgehenden Stieles befestigt und in einer entsprechenden Pfanne im Kasten des Femurteils in Art eines Gelenkkopfes aufgenommen ist. Eine solche Kniegelenkendoprothese ist aus der EP 0 639 358 oder aus der GB-A-2,088,724 bekannt. Das Problem bei einer solchen Kugelkonstruktion besteht im Einhängen der Kugel in die Pfanne und in der latenten Gefahr einer Luxation bei Zug- oder Extrembewegungen. Der Gefahr der Luxation soll teilweise dadurch begegnet werden, daß der Stiel, auf dessen Spitze die Kugel befestigt ist, nicht fest mit dem Tibiateil verbunden, sondern in einer Ausnehmung in dem Tibiaschaft eingelassen war, wodurch bei Zugbeanspruchung nicht das Kugelgelenk aus der Pfanne, sondern der Stiel aus der Ausnehmung gezogen wird, was wegen der großen Weglänge ohne Bedeutung ist.

Aus der DE-OS 41 02 509 ist eine neuere Konstruktion einer Kniegelenkendoprothese mit einem Scharniergelenk bekannt, bei der eine festsitzende Kugel auf einem Stiel und eine am Femurteil befestigte, geschlitzte Pfanne vorgesehen ist. Anhand dieser bekannten Konstruktion wird das Problem des Einhängens und der Luxation offensichtlich.

Schließlich ergibt sich ein weiteres Problem bei all diesen Kugelkonstruktionen aus der Tatsache, daß die wesentlichsten auf den Kupplungsbolzen einwirkenden Kräfte pendelnde, anteriore/posteriore Schubkräfte sind, die von den seitlichen, an die Kastenwand artikulierenden Kugelkalotten nur ganz unbedeutend neutralisiert werden können, wodurch die tragenden Flächen auf die zentralen Anteile der Kugel bzw. der Pfanne reduziert werden und dementsprechend die Gefahr einer Überlastung besteht.

Aufgabe der vorliegenden Erfindung ist es demgegenüber, eine Kniegelenkendoprothese der eingangs genannten Art derart weiterzubilden, daß die geschilderten Nachteile vermieden werden und insbesondere eine einfache Möglichkeit einer Varus/Valguskompensation besteht.

Diese Aufgabe wird bei der Kniegelenkendoprothese der eingangs genannten Art erfindungsgemäß durch vier unterschiedliche Ausführungsformen gelöst. In einer ersten Ausführungsform ist vorgesehen, daß der sich innerhalb der Bohrung des Gelenkkopfes befindliche Abschnitt des Kupplungsbolzens eine sphärisch geformte Außenmantelfläche aufweist, die mit einer entsprechend geformten Gleitfläche des Gelenkkopfes derart zusammenwirkt, daß sowohl eine Flexions-/Extensionsbewegung als auch eine Varus-/Valgusbewegung zwischen dem Tibiateil (2) und dem Femurteil (1) möglich ist.

Die Vorteile dieser erfindungsgemäßen Ausführungsform liegen insbesondere darin, daß der die sphärisch geformte Achse umgebende ringförmige Gelenkkopf bis zum Anschlag an den Seitenwänden der Kondylenschalen in Varus- oder Valgusrichtung gekippt oder parallel zur Tibiaachse gedreht werden kann.

Die zweite erfindungsgemäße Ausführungsform sieht vor, daß der sich innerhalb der Bohrung des Gelenkkopfes befindliche Abschnitt des Kupplungsbolzens die Form eines Zylinders aufweist, dessen Achse quer zur Längsachse des Kupplungsbolzens verläuft, wobei der Zylinder mit einer entsprechend geformten Gleitfläche innerhalb des Gelenkkopfes zusammenwirkt, und daß der Kupplungsbolzen an den Seitenwänden der Kondylenschalen schwenkbar gelagert ist, um eine Flexions-/Extensionsbewegung zu ermöglichen. Die Achse des Zylinders verläuft somit in anteriorer/posteriorer Richtung. Auch bei dieser Ausführungsform bestehen die Vorteile in der Beweglichkeit des Gelenkkopfes in Varus- oder Valgusrichtung, während hier allerdings eine Rotation um die Tibiaachse bzw. parallel dazu nicht möglich ist.

Bei der beschriebenen ersten und zweiten Ausführungsform kann die Gleitfläche des Gelenkkopfes aus einem zum einen gleitfähigen und zum anderen widerstandsfähigen Material bestehen. Dieses Material ist vorzugsweise Kunststoff oder dasselbe Material, aus dem der Kupplungsbolzen besteht (z.B. Metall/Metall; Keramik/Keramik usw.). Sowohl im einen wie im anderen Fall kann das entsprechende Material, sofern es nicht ohnehin identisch ist mit jenem des Gelenkkopfes, in Form einer Lagerhülse in den Gelenkkopf eingelassen sein.

Die beiden weiteren erfindungsgemäßen Ausführungsformen machen sich das Prinzip der beiden vorstehend beschriebenen Ausführungsformen zunutze, beinhalten jedoch eine auf den Kupplungsbolzen aufgesteckte Gleithülse, die in der Bohrung des Gelenkkopfes sitzt, und die sowohl eine Flexions-/Extensionsbewegung als auch eine Varus-/Valgusbewegung zwischen dem Tibiateil und dem Femurteil der Kniegelenkendoprothese ermöglicht.

Gemäß der dritten erfindungsgemäßen Ausführungsform ist somit vorgesehen, daß die Gleithülse eine sphärisch geformte Außenmantelfläche aufweist, die mit einer entsprechend geformten Gleitfläche des Gelenkkopfes zusammenwirkt. Auch hier kann der die sphärische Gleithülse umgebende ringförmige Gelenkkopf bis zum Anschlag an den Seitenwänden der Kondylenschalen in Varusoder Valgusrichtung gekippt oder in beschränktem Ausmaß parallel zur Tibiaachse gedreht werden. Um die Flexions/Extensionsbewegung ausschließlich zwischen der Außenmantelfläche der Gleithülse und der entsprechenden Gleitfläche des Gelenkkopfes stattfinden zu lassen, kann vorgesehen werden, daß die Gleithülse drehfest mit dem Kupplungsbolzen verbunden ist.

Schließlich sieht die vierte erfindungsgemäße Ausführungsform vor, daß die Gleithülse die Form eines Zylinders aufweist, dessen Längsachse quer zur Achse der Bohrung des Gelenkkopfes verläuft, wobei auch hier die zylindrische Gleithülse eine ventral-dorsale Ausrichtung besitzt und mit einer entsprechend geformten Gleitfläche innerhalb des Gelenkkopfes zusammenwirkt.

Der Vorteil bei der Verwendung der Gleithülsen liegt darin, daß die Reibung zwischen dem Kupplungsbolzen und der Gleitfläche des Gelenkkopfes vermindert wird. Darüber hinaus ermöglichen die Gleithülsen eine Aufteilung der Gelenkfläche in einen rein zylindrischen Part für die Flexions-/Extensionsbewegung zwischen dem Kupplungsbolzen und der Gleithülse und in einen sphärischen bzw. weiteren zylindrischen Part für die Varus/Valgusbewegung zwischen Gleithülse und Gelenkkopf, wobei der sphärische Part der Gelenkfläche auch noch eine eingeschränkte Rotationsbewegung zwischen Gleithülse und Gelenkkopf ermöglicht.

Die Gleithülse ihrerseits kann aus einheitlichem Material bestehen, vorzugsweise aus Kunststoff, Metall oder Keramik. Sie kann aber auch an den Gleitflächen ein anderes Material aufweisen. So ist beispielsweise denkbar, daß die konvex geformte Außenfläche aus einem harten Material besteht, z.B. Metall oder Keramik, während die konkav geformte innere Gleitfläche aus einem weichen Material, z.B. Kunststoff, besteht, welches gemäß einer Weiterbildung der vorliegenden Erfindung in Form einer Lagerhülse in die Gleithülse eingelassen sein kann.

Die Vorteile aller vier Ausführungsformen der Erfindung liegen in einer besonders haltbaren, stabilen Konstruktion des Kniegelenks. Indem nämlich nur der zentrale kugelförmige bzw. zylindrische Teil des Kupplungsbolzens als Gelenkachse verwendet wird und der Kupplungsbolzen ansonsten fest mit den beiden Seitenwänden der Kondylenscheiben verbunden ist, entfallen die aus dem Stand der Technik bekannten nichttragenden seitlichen Kugelkalotten. Deshalb kann der zentrale kugelförmige bzw. zylindrische Teil des Kupplungsbolzens größer und belastungssicherer dimensioniert werden, was insgesamt eine höhere Bruchsicherheit bzw. eine größere Belastbarkeit der erfindungsgemäßen Kniegelenkendoprothese zur Folge hat.

Vorteilhafte Weiterbildungen der Erfindung sind in den Unteransprüchen angegeben.
Für die vorstehend beschriebenen Ausführungsformen 1, 3 und 4 ist vorzugsweise vorgesehen, daß der Kupplungsbolzen an den angrenzenden Seitenwänden der Kondylenschalen drehfest gelagert, d.h. fest mit den Seitenwänden verbunden ist. Der Vorteil dieser bevorzugten Möglichkeit der Halterung des Kupplungsbolzens besteht darin, daß die Gefahr einer Dislokation weitestgehend vermieden wird. Grundsätzlich ist es aber bei allen vier erfindungsgemäßen Ausführungsformen möglich, den Kupplungsbolzen schwenkbar in den Seitenwänden der Kondylenschalen zu lagern.

Der Parallelabstand der Seitenwände der Kondylenschalen ist vorzugsweise derart bemessen, daß die Varus-/Valgusbeweglichkeit auf einen vorgebbaren Winkelbereich γ beschränkt ist, der beispielsweise zwischen 0° und ± 10° liegt.

Zur Verminderung des Verschleißes zwischen den Seitenflächen des Gelenkkopfes und den Seitenwänden der Kondylenschalen ist dort jeweils eine Scheibe angeordnet, die beispielsweise aus Polyäthylen bestehen kann.

Vorzugsweise sind die Seitenflächen des Gelenkkopfes in radialer Richtung beidseits konvex-konisch ausgebildet, wodurch bewirkt wird, daß ein Anschlag der Seitenflächen an den Seitenwänden der Kondylenschalen bzw. an der dazwischen angeordneten Scheibe flächig ausfällt und dadurch bei gleichzeitiger Flexions-/Extensionsbewegung keine durch Kantendruck erzeugten Rillen eingeschliffen werden.

Überdies kann durch teilkonische (dachförmige) Anschrägung der Seitenflächen des Gelenkkopfes die Rotation des Gelenkkopfes ausgeschaltet werden, die überflüssig ist, da die notwendige Rotation um die Achse des Kupplungszapfens ablaufen kann. Schließlich ist es auch möglich, durch entsprechende konische Ausbildung der zum Gelenkkopf weisenden Innenseiten der Scheiben die Varus-/Valgusbewegung und die Rotationsbewegung einzuschränken.

Im folgenden werden die dritte und vierte Ausführungsform der erfindungsgemäßen Kniegelenkendoprothese anhand einer Zeichnung näher erläutert.

Es zeigen:
- Fig. 1: eine Explosionsdarstellung einer Kniegelenkendoprothese mit einer sphärischen Gleithülse im Kupplungsteil;
- Fig. 2: einen Vertikalschnitt durch das Femurteil mit montiertem Kupplungsteil gemäß Fig. 1, wobei die linke Hälfte des Gelenkkopfes der ersten und zweiten Ausführungsform der Erfindung entspricht, während die rechte Hälfte des Gelenkkopfes der dritten bzw. vierten Ausführungsform der Erfindung mit Gleithülse entspricht; und
- Fig. 3: eine perspektivische Skizze einer zylindrischen Gleithülse.

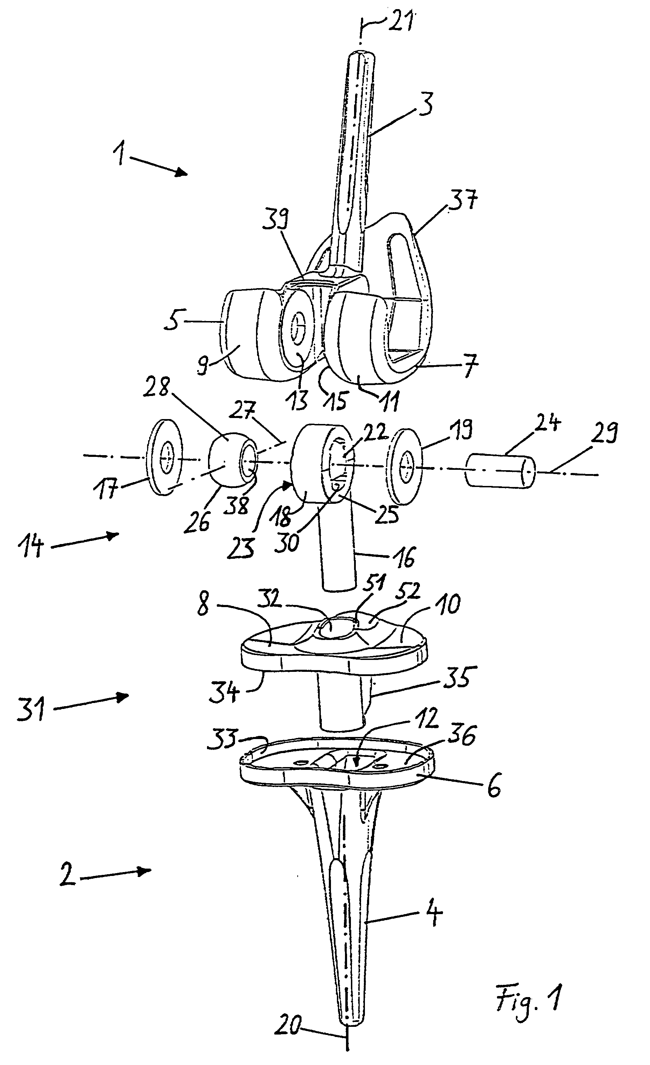

Fig. 1 zeigt eine Explosionsdarstellung einer Kniegelenkendoprothese, die im wesentlichen aus vier Baugruppen besteht: einem Femurteil 1 mit einem Femurschaft 3 und an dessen unterem Ende angeordneten konvex gekrümmten Kondylenschalen 5, 7, welche den natürlichen Kondylen weitgehend angepaßt sind und auf ihrer Unterseite erste Gleitflächen 9, 11 aufweisen, und die dorsal zwei voneinander beabstandete Seitenwände 13, 15 aufweisen, die mit einem Steg zu einem Kasten 39 verbunden sind und innerhalb des Kastens 39 einen interkondylären Zwischenraum bilden. Die beiden Kondylenschalen 5, 7 gehen ventral ineinander über und bilden dort ein Patellaschild 37 zur Artikulation mit der Patella.

Das Pendant zum Femurteil 1 ist ein Tibiateil 2 mit einem Tibiaschaft 4 und einem Tibiaplateau 6 an dessen oberem Ende, welches eine Tragfläche 36 aufweist, die eine sich axial in dem Tibiaschaft 4 erstreckende Ausnehmung 12 sowie ein Seitenbord 33 zur Aufnahme einer dritten Baugruppe 31 aufweist.

Diese dritte Baugruppe 31 besteht aus einem Inlay mit einer Bodenfläche 34 zur Auflage auf der Tragfläche 36 des Tibiateils 2, mit einem an der Bodenfläche 34 ansetzenden Hülsenkörper 35, der zur Befestigung des Inlays 31 an dem Tibiateil 2 in die Ausnehmung 12 des Tibiateils 2 gesteckt wird, ferner mit zweiten Gleitflächen 8, 10 auf der Oberseite 52 des Inlays 31, welche in ventral-dorsaler Richtung konkav gekrümmt sind und auf denen sich die Kondylenschalen 5, 7 des Femurteils 1 abstützen. Zur Befestigung der vierten Baugruppe, dem Kupplungsteil 14, an dem Tibiateil 2 weist das Inlay 31 eine Bohrung 32 auf, welche sich durch die Oberseite 52 des Inlays und durch den Hülsenkörper 35 erstreckt.

Das Kupplungsteil 14 weist als zentrales Element einen Kupplungszapfen 16 und an dessen oberem Ende einen in den interkondylären Zwischenraum des Femurteils 1 hineinragenden Gelenkkopf 18 auf. Im zusammengebauten Zustand der Kniegelenkendoprothese ist der Kupplungszapfen 16 in der Bohrung 32 des Inlays 31 und damit in der Ausnehmung 12 des Tibiateils 2 drehbar aufgenommen. Der Gelenkkopf 18 besitzt eine quer zur Tibiaachse 20 bzw. quer zur Femurachse 21 verlaufende Bohrung 22 und ist mittels eines Kupplungsbolzens 24 um eine im wesentlichen horizontale Gelenkachse 29 schwenkbar in den Seitenwänden 13, 15 der Kondylenschalen 5, 7 gelagert. Die Bohrung 22 in dem Gelenkkopf 18 weist eine Gleitfläche 30 auf, die derart geformt ist, daß sie einen Lagersitz für eine Gleithülse 26 bildet, die im zusammengesetzten Zustand der Kniegelenkendoprothese zwischen dem Kupplungsbolzen 24 und dem Gelenkkopf 18 angeordnet, d.h. mit ihrer Bohrung 38 auf den Kupplungsbolzen 24 aufgesteckt ist. In der in Fig. 1 dargestellten Ausführungsform weist die Gleithülse eine sphärische Außenmantelfläche 28 auf, die mit der entsprechend geformten Gleitfläche 30 des Gelenkkopfes 18 zusammenwirkt und eine Aufteilung der Gelenkflächen auf den zylindrischen Kupplungsbolzen 24 und die sphärische Gleithülse 26 ermöglicht: der zylindrische Kupplungsbolzen 24 übernimmt ausschließlich die Flexions-/Extensionsbewegung zwischen dem Kupplungsbolzen und der Gleithülse, während die sphärische Gleithülse 26 sowohl die Varus-/Valgusbewegung als auch eine Rotationsbewegung zwischen Gleithülse und Gelenkkopf übernimmt. Zur Verschleißminderung zwischen den Seitenflächen 23, 25 des Gelenkkopfes 18 einerseits und den dagegen drehenden Seitenwänden 13, 15 der Kondylenschalen 5, 7 andererseits sind Scheiben 17, 19 vorgesehen, die ebenfalls auf den Kupplungsbolzen 24 aufgesteckt sind.

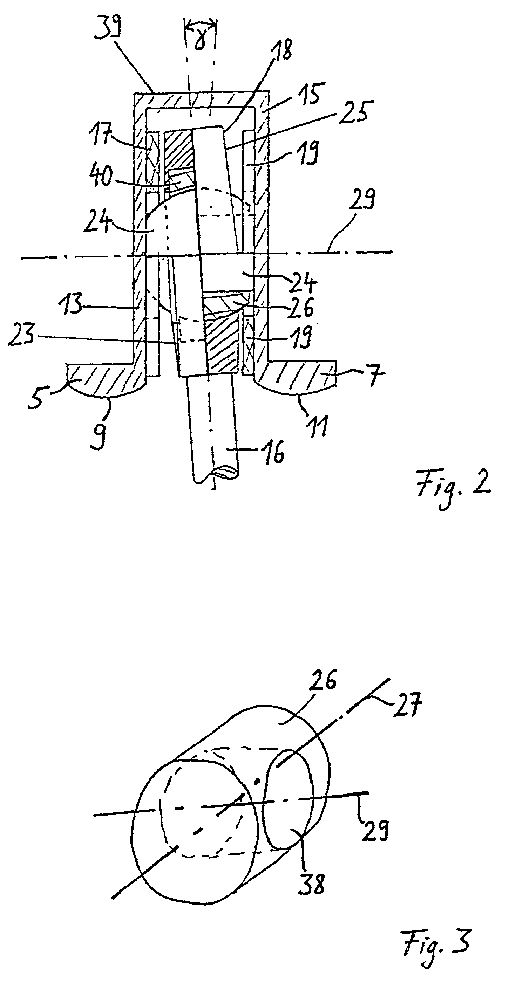

Fig. 2 zeigt einen Vertikalschnitt durch das Femurteil 1 mit montiertem Kupplungsteil 14, mit vier unterschiedlichen Ausführungsformen des sich innerhalb der Bohrung 22 des Gelenkkopfes 18 befindlichen zentralen Mittelabschnitts des Kupplungsbolzens 24. In der linken Häfte der Schnittdarstellung besitzt der Mittelabschnitt eine sphärische oder eine senkrecht zur Achse 29 zylindrische Form und ist von einer entsprechend geformten Lagerhülse 40 umgeben, die in die Bohrung 22 des Gelenkkopfes 18 eingelassen ist und aus Kunststoff besteht. In der rechten Hälfte der Schnittdarstellung ist der Mittelabschnitt des Kupplungsbolzens 24 in Form eines Zylinders um seine Längsachse 29 ausgebildet und ist von einer sphärisch oder senkrecht zur Achse 29 zylindrisch geformten Gleithülse 26 umgeben, die mit einer entsprechend geformten Gleitfläche 30 des Gelenkkopfes 18 zusammenwirkt. Auch die Innengleitfläche der Gleithülse 26 kann aus Kunststoff bestehen, welcher in Form einer Lagerhülse in die Gleithülse eingelassen ist. Die Außenmantelfläche der Gleithülse würde dann aus härterem Material, z.B. Metall oder Keramik, bestehen. Alternativ hierzu kann die Gleithülse aus einheitlichem Material bestehen, bevorzugterweise aus Kunststoff, Metall oder Keramik.

Die Seitenwände 13, 15 der Kondylenschalen 5, 7 sind durch einen oberen Steg zu einem Kasten 39 verbunden, wobei der Parallelabstand der Seitenwände 13, 15 derart bemessen ist, daß die Varus-/Valgusbeweglichkeit auf einen Winkelbereich γ von etwa ± 10° beschränkt ist. Durch entsprechende konische Gestaltung der Seitenflächen 23, 25 des Gelenkkopfes 18 liegt der Gelenkkopf bei extremer Varus-/Valgusauslenkung flächig an den Scheiben 17, 19 an. Im unteren Bereich sind die Seitenflächen 23, 25 des Gelenkkopfes 18 nochmals konisch angeschrägt, um eine nicht nötige Rotation des Gelenkkopfes auszuschließen.

Fig. 3 zeigt eine perspektivische Skizze einer zylindrischen Gleithülse 26 mit einer ventral-dorsal oder anterior-posterior ausgerichteten Längsachse 27 und einer Bohrung 38 zum Durchstecken des Kupplungsbolzens 24. Selbstverständlich kann auch der Kupplungsbolzen 24 in seinem zentralen Abschnitt, welcher sich im zusammengebauten Zustand der Kniegelenkendoprothese innerhalb der Bohrung 22 des Gelenkkopfes 18 befindet, eine wie in Fig. 3 dargestellte zylindrische Form mit gleicher Ausrichtung der Längsachse 27 aufweisen. Die zylindrische Gleithülse 26 bzw. der zylindrische zentrale Abschnitt des Kupplungsbolzens 24 ist in der entsprechend geformten Gleitfläche 30 des Gelenkkopfes 18 aufgenommen, welcher als Sitz für die Gleithülse 26 bzw. den zentralen Mittelabschnitt des Kupplungsbolzens 24 dient. Auch mit dieser Ausführungsform ist bei der Indikation von axialen Fehlstellungen eine Varus-/Valguskompensation zusätzlich zur Flexions-/Extensionsbewegung möglich.

## Patentansprüche

1. Kniegelenkendoprothese, mit
einem Femurteil (1), das am unteren Ende eines Femurschaftes (3) konvex gekrümmte Kondylenschalen (5, 7) mit ersten Gleitflächen (9, 11) aufweist, die dorsal zwei voneinander beabstandete, einen interkondylären Zwischenraum bildende Seitenwände (13, 15) besitzen;
einem Tibiateil (2), das am oberen Ende eines Tibiaschaftes (4) ein Tibiaplateau (6) aufweist, auf dem den ersten Gleitflächen (9, 11) der Kondylenschalen (5, 7) entsprechende zweite Gleitflächen (8, 10) ausgebildet sind, und das eine sich axial in dem Tibiaschaft (4) erstreckende Ausnehmung (12) aufweist; und mit
einem Kupplungsteil (14), das am oberen Ende eines drehbar in der Ausnehmung (12) aufgenommenen Kupplungszapfens (16) einen in den interkondylären Zwischenraum hineinragenden Gelenkkopf (18) aufweist, der eine quer zur Femurachse (21) verlaufende Bohrung (22) besitzt und mittels eines durch die Bohrung laufenden und sich an den angrenzenden Seitenwänden (13, 15) der Kondylenschalen (5, 7) abstützenden Kupplungsbolzens (24) schwenkbar gelagert ist,
**dadurch gekennzeichnet, daß**
der sich innerhalb der Bohrung (22) des Gelenkkopfes (18) befindliche Abschnitt des Kupplungsbolzens (24) eine sphärisch geformte Außenmantelfläche aufweist, die mit einer entsprechend geformten Gleitfläche (30) des Gelenkkopfes (18) derart zusammenwirkt, daß sowohl eine Flexions-/Extensionsbewegung als auch eine Varus-/Valgusbewegung zwischen dem Tibiateil (2) und dem Femurteil (1) möglich ist.

2. Kniegelenkendoprothese nach dem Oberbegriff des Anspruchs 1,
**dadurch gekennzeichnet, daß**
der sich innerhalb der Bohrung (22) des Gelenkkopfes (18) befindliche Abschnitt des Kupplungsbolzens (24) die Form eines Zylinders aufweist, dessen Achse quer zur Achse des Kupplungsbolzens (24) verläuft, und der mit einer entsprechend geformten Gleitfläche (30) des Gelenkkopfes (18) zusammenwirkt, und daß der Kupplungsbolzen (24) an den angrenzenden Seitenwänden (13, 15) der Kondylenschalen (5, 7) schwenkbar gelagert ist.

3. Kniegelenkendoprothese nach dem Oberbegriff des Anspruchs 1,
**dadurch gekennzeichnet, daß**
in der Bohrung (22) des Gelenkkopfes (18) eine auf den Kupplungsbolzen (24) aufgesteckte Gleithülse (26) sitzt, welche sowohl eine Flexions-/Extensionsbewegung als auch eine Varus-/Valgusbewegung zwischen dem Tibiateil (2) und dem Femurteil (1) ermöglicht.

4. Kniegelenkendoprothese nach Anspruch 3,
**dadurch gekennzeichnet, daß**
die Gleithülse (26) eine sphärisch geformte Außenmantelfläche (28) aufweist, die mit einer entsprechend geformten Gleitfläche (30) des Gelenkkopfes (18) zusammenwirkt (Fig. 1).

5. Kniegelenkendoprothese nach Anspruch 4,
**dadurch gekennzeichnet, daß**
die Gleithülse (26) drehfest mit dem Kupplungsbolzen (24) verbunden ist.

6. Kniegelenkendoprothese nach Anspruch 3,
**dadurch gekennzeichnet, daß**
die Gleithülse (26) die Form eines Zylinders aufweist, dessen Achse (27) quer zur Achse (29) der Bohrung (22) des Gelenkkopfes (18) verläuft, und der mit einer entsprechend geformten Gleitfläche (30) des Gelenkkopfes (18) zusammenwirkt (Fig. 2).

7. Kniegelenkendoprothese nach einem der Ansprüche 3 bis 6,
**dadurch gekennzeichnet, daß**
die Außenmantelfläche (28) der Gleithülse (26) aus einem harten Material wie Metall oder Keramik besteht, während die Innengleitfläche der Gleithülse (26) aus weichem Material wie Kunststoff besteht und in Form einer Lagerhülse in die Gleithülse (26) eingelassen ist.

8. Kniegelenkendoprothese nach einem der Ansprüche 1 oder 3 bis 7,
**dadurch gekennzeichnet, daß**
der Kupplungsbolzen (24) an den angrenzenden Seitenwänden (13, 15) der Kondylenschalen (5, 7) drehfest gelagert ist.

9. Kniegelenkendoprothese nach einem der vorstehenden Ansprüche,
**dadurch gekennzeichnet, daß**
der Parallelabstand der Seitenwände (13, 15) der Kondylenschalen (5, 7) derart bemessen ist, daß die Varus-/Valgusbeweglichkeit auf einen vorgebbaren Winkelbereich γ beschränkt ist.

10. Kniegelenkendoprothese nach Anspruch 9,
**dadurch gekennzeichnet, daß**
der Winkelbereich zwischen 0° und ± 10° liegt.

11. Kniegelenkendoprothese nach einem der vorstehenden Ansprüche,
**dadurch gekennzeichnet, daß**
zwischen dem Gelenkkopf (18) und den Seitenwänden (13, 15) der Kondylenschalen (5, 7) je eine Scheibe (17, 19) angeordnet ist.

12. Kniegelenkendoprothese nach einem der vorstehenden Ansprüche,
**dadurch gekennzeichnet, daß**
die Seitenflächen (23, 25) des Gelenkkopfes (18) in radialer Richtung beidseits konvex konisch ausgebildet sind.

13. Kniegelenkendoprothese nach einem der vorstehenden Ansprüche,
**dadurch gekennzeichnet, daß**
die Seitenflächen (23, 25) des Gelenkkopfes (18) proximal/distal dachförmig angeschrägt und anterior/posterior plan ausgebildet sind.

14. Kniegelenkendoprothese nach einem der Ansprüche 11 bis 13,
**dadurch gekennzeichnet, daß**
die Scheiben (17, 19) auf der zum Gelenkkopf (18) weisenden Innenseite konkav konisch ausgebildet sind.

## Claims

1. A stifle joint endoprosthesis including
a femur piece (1) comprising at the lower end of a femur shaft (3) convexly curved condylar cups (5, 7) having first sliding surfaces (9, 11), which dorsally possess two side walls (13, 15) spaced apart from each other and forming an intercondylar interspace (13, 15);
a tibia piece (2) that comprises at the upper end of a tibia shaft (4) a tibia plane (6), on which second sliding surfaces (8, 10) are formed corresponding to the first sliding surfaces (9, 11) of the condylar cups (5, 7), and that comprises a recess (12) extending radially within said tibia shaft (4); and including
a coupling piece (14) that on the upper end of a pivotably mounted coupling pin (16) received in said recess (12) comprises an articulation head (18) protruding into said intercondylar interspace, which articulation head (18) possesses a bore (22) running transversely to the femur axis (21) and is pivotably mounted by means of a coupling pin (24) supporting on the adjacent side walls (13, 15) of the condylar cups (5, 7),
**characterized in that**
the portion of the coupling pin (24) situated inside of bore (22) of articulation head (18), comprises a spherically formed outer envelope that cooperates with a correspondingly formed sliding surface (30) of the articulation head (18) in such a manner that a flexion/extension movement, as well as a varus/valgus movement between the tibia piece (2) and the femur piece (1) is possible.

2. The stifle joint endoprosthesis according to the preamble of claim 1,
**characterized in that**
the portion of the coupling pin (24) situated inside of bore (22) of the articulation head (18) has the form of a cylinder, the axis of which runs transversely to the axis of the coupling pin (24) and which cooperates with a correspondingly formed sliding surface (30) of articulation head (18), and that said coupling pin (24) is pivotably mounted to the adjacent side walls (13, 15) of the condylar cups (5, 7).

3. The stifle joint endoprosthesis according to the preamble of claim 1,
**characterized in that**
in the bore (22) of the articulation head (18), a sliding sleeve (26) slipped on said coupling pin (24) is seated, which sliding sleeve (26) enables a flexion/extension movement as well as a varus/valgus movement between said tibia piece (2) and said femur piece (1).

4. The stifle joint endoprosthesis according to claim 3,
**characterized in that**
the sliding sleeve (26) has a spherically formed outer envelope (28) cooperating with a correspondingly formed sliding surface (3) of the articulation head (18) (Fig. 1).

5. The stifle joint endoprosthesis according to claim 3,
**characterized in that**
the sliding sleeve (26) is connected non-rotatably to the coupling pin (24).

6. The stifle joint endoprosthesis according to claim 3,
**characterized in that**
the sliding sleeve (26) has the form of a cylinder, the axis (27) of which runs transversely to the axis (29) of bore (22) of the articulation head (18), and which cooperates with a correspondingly formed sliding surface (30) of articulation head (18) (Fig. 2).

7. The stifle joint endoprosthesis according to any one of claims 3 through 6,
**characterized in that**
the outer envelope (28) of the sliding sleeve (26) is comprised of a hard material such as metal or ceramics, whereas the inner sliding surface of the sliding sleeve (26) is comprised of a soft material such as plastic, and is recessed in said sliding sleeve (26) in the form of a bearing sleeve.

8. The stifle joint endoprosthesis according to any one of claims 1 or 3 through 7,
**characterized in that**
the coupling pin (24) is non-rotatably mounted on the adjacent side walls (13, 15) of the condylar cups (5, 7).

9. The stifle joint endoprosthesis according to any one of the preceding claims,
**characterized in that**
the parallel spacing of the side walls (13, 15) of the condylar cups (5, 7) is dimensioned in such a manner that the varus/valgus mobility is restricted to a pre-definable angular range γ.

10. The stifle joint endoprosthesis according to claim 9,
**characterized in that**
the angular range is between 0° and ± 10°.

11. The stifle joint endoprosthesis according to any one of the preceding claims 6,
**characterized in that**
between the articulation head (18) and the side walls (13, 15) of the condylar cups (5, 7) a disk (17, 19) is in each case arranged.

12. The stifle joint endoprosthesis according to any one of the preceding claims,
**characterized in that**
the side faces (23, 25) of the articulation head (18) are bilaterally configured convexly conical in the radial direction.

13. The stifle joint endoprosthesis according to any one of the preceding claims,
**characterized in that**
the side faces (23, 25) of the articulation head (18) are configured proximally/distally inclined in a roof-shape and anteriorly/posteriorly planar.

14. The stifle joint endoprosthesis according to any one of the claims 11 through 13,
**characterized in that**
the disks (17, 19) at the inner side facing the articulation head (18) are configured concavely conical.

## Revendications

1. Endoprothèse pour l'articulation du genou, comprenant une pièce fémur (1) présentant sur l'extrémité inférieure d'une tige fémur (3) des coupes condyliennes (5, 7) courbées de manière convexe, ayant des premières surfaces de glissement (9, 11) possédant en position dorsale deux parois latérales (13, 15) écartées l'une de l'autre et formant un espace intermédiaire inter-condylien ;
une pièce tibia (2) présentant sur l'extrémité supérieure d'une tige tibia (4) un plateau tibia (6), sur lequel sont formées des secondes surfaces de glissement (8, 10) correspondant aux premières surfaces de glissement (9, 11) des coupes condyliennes (5, 7), et ladite pièce tibia (2) présentant une cavité (12) s'étendant de façon axiale dans la tige tibia (4) ; et
comprenant une pièce d'accouplement (14) qui présente sur l'extrémité supérieure d'un pivot d'accouplement (16) reçu dans ladite cavité (12), une tête d'articulation (18) se projetant à l'intérieur dudit espace intermédiaire inter-condylien, et qui possède un alésage (22) passant en travers de l'axe fémur (21), et qui est logée de manière pivotante au moyen d'un tourillon d'accouplement (24) passant par ledit alésage et s'appuyant contre les parois latérales (13, 15) adjacentes des coupes condyliennes (5, 7),
**caractérisée en ce que**
la partie du tourillon d'accouplement (24) située à l'intérieur de l'alésage (22) de la tête d'articulation (18) présente une enveloppe extérieure d'une forme sphérique coopérant avec la surface de glissement (30) de forme correspondante de manière à ce que non seulement un mouvement de flexion / extension, mais aussi un mouvement varus / valgus entre la pièce tibia (2) et la pièce fémur (1) soit possible.

2. Endoprothèse pour l'articulation du genou selon le préambule de la revendication 1,
**caractérisée en ce que**
la partie du tourillon d'accouplement (24) située à l'intérieur de l'alésage (22) de la tête d'articulation (18) présente la forme d'un cylindre dont l'axe passe en travers de l'axe du tourillon d'accouplement (24), et qui coopère avec une surface de glissement (30) de forme correspondante de la tête d'articulation (18), et **en ce que** le tourillon d'accouplement (24) est logé de manière pivotante sur les parois latérales (13, 15) des coupes condyliennes (5, 7).

3. Endoprothèse pour l'articulation du genou selon le préambule de la revendication 1,
**caractérisée en ce**
**qu'**une douille glissante (26) mise sur le tourillon d'accouplement (24), est siégée dans l'alésage (22) de la tête d'articulation (18), ladite douille glissante (26) permettant non seulement un mouvement de flexion / extension, mais aussi un mouvement varus / valgus entre la pièce tibia (2) et la pièce fémur (1).

4. Endoprothèse pour l'articulation du genou selon la revendication 3,
**caractérisée en ce que**
ladite douille de glissement (26) présente une enveloppe extérieure (28) de forme sphérique, qui coopère avec une surface de glissement (30) de forme correspondante de la tête d'articulation (18) (Fig. 1).

5. Endoprothèse pour l'articulation du genou selon la revendication 4,
**caractérisée en ce que**
ladite douille de glissement (26) est liée solidairement en rotation avec le tourillon d'accouplement (24).

6. Endoprothèse pour l'articulation du genou selon la revendication 3,
**caractérisée en ce que**
ladite douille de glissement (26) présente la forme d'un cylindre dont l'axe (27) passe en travers de l'axe (29) de l'alésage (22) de la tête d'articulation (18), et qui coopère avec une surface de glissement (30) de forme correspondante de la tête d'articulation (18) (Fig. 2).

7. Endoprothèse pour l'articulation du genou selon l'une quelconque des revendications 3 à 6,
**caractérisée en ce que**
ladite enveloppe extérieure (28) de la douille de glissement (26) est constituée d'un matériau dur comme le métal ou la céramique, tandis que la surface de glissement intérieure de la douille de glissement (26) est constituée d'un matériau souple comme une matière synthétique, et est encastrée dans ladite douille de glissement (26) sous la forme d'une douille de support.

8. Endoprothèse pour l'articulation du genou selon l'une quelconque des revendications 1 ou 3 à 7,
**caractérisée en ce que**
ledit tourillon d'accouplement (24) est logé solidairement en rotation sur les parois latérales (13, 15) des coupes condyliennes (5, 7).

9. Endoprothèse pour l'articulation du genou selon l'une quelconque des revendications précédentes,
**caractérisée en ce que**
l'écart parallèle des parois latérales (13, 15) des coupes condyliennes (5, 7) est dimensionné de manière à limiter la mobilité varus / valgus à un rayon angulaire γ qui peut être prédéterminé.

10. Endoprothèse pour l'articulation du genou selon la revendication 9,
**caractérisée en ce que**
ledit rayon angulaire est compris entre 0° et ± 10°.

11. Endoprothèse pour l'articulation du genou selon l'une quelconque des revendications précédentes,
**caractérisée en ce que**
entre la tête d'articulation (18) et les parois latérales (13, 15) des coupes condyliennes (5, 7) un disque (17, 19) est disposé entre chaque.

12. Endoprothèse pour l'articulation du genou selon l'une quelconque des revendications précédentes,
**caractérisée en ce que**
les faces latérales (23, 25) de la tête d'articulation (18) sont bilatéralement configurées de façon convexe et conique en direction radiale.

13. Endoprothèse pour l'articulation du genou selon l'une quelconque des revendications précédentes,
**caractérisée en ce que**
les faces latérales (23, 25) de la tête d'articulation (18) sont configurées inclinées en position proximale / distale en forme d'un toit, et planaires en position antérieure / postérieure.

14. Endoprothèse pour l'articulation du genou selon l'une quelconque des revendications 11 à 13,
**caractérisée en ce que**
les disques (17, 19) sur la face intérieure orientée vers la tête d'articulation (18) sont configurés de façon concave et conique.
